# EUROPEAN PATENT SPECIFICATION

(11) **EP 2 747 772 B1**
(45) Date of publication and mention of the grant of the patent: **05.08.2015**
(21) Application number: 12762365.0
(22) Date of filing: 24.08.2012
(51) Int. Cl.: A61K 31/79, A61K 33/18, A61K 47/38, A61K 9/00, A61K 9/08, A61K 9/12, A61P 31/00, A61P 31/04, A61P 31/10

(54) **OPHTHALMIC FORMULATIONS**
OPHTHALMIKUM
FORMULATION OPHTALMIQUE

(30) Priority: 25.08.2011 GB 201114725
(43) Date of publication of application: 02.07.2014
(73) Proprietor: Altacor Limited, Cambridge, Cambridgeshire CB4 0WS (GB)
(72) Inventor: CRAWFORD, Francesca, Cambridge CB4 0WS (GB); GUNNING, Philip, Cambridge CB4 0WS (GB)
(74) Representative: Teuten, Andrew John
(86) International application number: PCT/GB2012/052082
(87) International publication number: WO 2013/027065

(56) References cited:
- EP-A1- 0 476 777
- US-A- 5 126 127

## Description

This invention relates to povidone-iodine formulations packaged for ophthalmic use.

Povidone-iodine (polyvinylpyrrolidone-iodine or PVP-I) preparations are indicated clinically for the prevention and treatment of surface infections, pre-operative and post-operative scrubbing, treatment of wounds, washing of hospital room personnel and pre-operative skin preparation of patients. Commercial products such as Betadine^{®} and Videne^{®} both contain 10% PVP-I, wherein the PVP-I used itself contains 10% iodine. Neither of these commercial formulations is recommended or suitable for use in the eye, but they have both been used in the eye as extemporaneous dilutions 50/50 with water or saline. In addition, a 5% w/v preparation of Betadine^{®} is available in some countries for ophthalmic uses. Formulations that have been sold as 10% solutions require dilution before use, often by nursing staff. This is both inconvenient and can lead to errors and possible contamination during the dilution process after the initial manufacture. In particular, if the solutions are over-diluted the concentration of available iodine may be below the required amount for effective germicidal activity while insufficient dilution may result in intolerability.

There have been reports of adverse reactions (Ghosh et al, Anaesthesia, 2006, 61, pages 1116-1129) with each of these commercial 10% formulations when used in the eye, e.g. intense burning sensations. These reactions have been attributed to excipients in the formulation particularly surfactants or highly acidic pH.

The efficacy of such formulations in reducing and/or eliminating bacterial colonies, fungal organisms and other infective organisms commonly found on or around the ocular surface (cornea, conjunctiva and palpebral fornices) and periocular region (eyelids, eyebrow and upper cheek area) has a direct relationship to the amount of iodine available to exert antimicrobial effects. A 5% PVP-I solution has approximately 0.5% available iodine and this is sufficient to reduce bacterial colonies by 91% and the number of species by 50% (Isenberg et al, Community Eye Health, 2003, 16 pages 30-31). In addition 5% has been found to be more efficacious than 1% PVPI (Ferguson et al, British Journal of Ophthalmology, 2003, 87, pages 163-167). Thus formulations which are capable of maintaining the reservoir of available iodine over the shelf life can be expected to retain their efficacy over this period.

US Patent No. 5849291, the disclosure of which is relates to an enzymatically generated iodine microbiocide for the inactivation of pathogenic organisms that are contaminants in or on sensitive biological materials such as the human eye.

International Patent Application No WO2007/106381, relates to a topical ophthalmic composition comprised of povidone-iodine 0.01% to 10% combined with a steroid or non-steroidal anti-inflammatory drug.

International Patent Application No WO2009/097123, relates to a device for the in-situ preparation of a pharmaceutical composition comprising povidone-iodine and a steroid or non-steroid anti-inflammatory useful in the treatment of acute viral infections of the eyelids and ocular surfaces of humans and other mammals.

US Patent No. 5232692, relates to a composition comprising an aqueous solution of povidone-iodine having a concentration from 5% to 0.1% for neonatal ophthalmic prophylactic.

US Patent No. 4364929, relates to a germicidal colloidal lubricating gel comprising 0.05-2% iodine and a gel forming colloid. Ophthalmic use of the gel is not contemplated.

US Patent No 4954351, relates to a pharmaceutical iodophor preparation having predictable microbicidal effectiveness and long duration of action comprising PVP-I, free iodine, a source of iodide ions and a source of iodate ions, the preparation having a ratio of available (titratable) iodine to iodide between 2:1 and 10:1, a pH between 5 and 6 and a free iodine concentration between 2 and 20 ppm, wherein the amount of iodate ion in the preparation is sufficient to maintain the free iodine concentration between 2 and 20 ppm for at least 12 months at 20°C.

US Patent No 5709851, relates to a stable pharmaceutical composition with reduced irritancy, the composition comprising an aqueous solution of elemental iodine and at least one organic substance which reacts with iodine, whereby iodine loss is controlled by providing a source of iodate ions in an amount sufficient to provide from 0.01% to 0.04% by weight iodate ions, preferably from 0.02% to 0.03% by weight iodate ions

US Patent No 5643608, relates to stable aqueous iodine/iodide/iodate germicidal compositions which have relatively high quantities of free iodine therein and also substantially maintain the starting amounts of available and free iodine throughout a storage period of at least about three months. The compositions of the invention contain from about 0.01-1.4% by weight available iodine, from about 10-125 ppm free iodine, from about 0.005-0.5% by weight iodate ion, from about 0.1-15% by weight of iodine complexing agent, from about 0.004-0.5% by weight iodide ion, and have a pH of from about 2.0-4.5

US Patent No 5178853, relates to an ophthalmic preparation comprising a povidone-iodine solution containing 0.01 to 0.1% of available iodine in a sterile primary packaging which consists of a bottle of glass type I or polyethylene-terephthalate with a dropper of polypropylene, polyethylene and/or polyfluoroethylene coated bromobutyl rubber. Said preparation presents an improved shelf-life. The povidone-iodine solution is prepared from povidone having a K-value of more than 30 and most preferably about 90.

US Patent No 4976969, describes an ophthalmic solution comprising 0.1 to 0.9 % volume of povidone-iodine in association with a suitable ophthalmic carrier, selected from the group consisting of hydroxyethyl cellulose, methylcellulose and polyvinylalcohol. The solution is useful in treating various ophthalmic disorders such as follicular conjunctivitis and giant papillary conjunctivitis resulting from reaction to contact lenses, cosmetics and allergies among others. It is also useful in the treatment of ocular dryness resulting from post menopausal stage and from Sjögren's disease.

US patent No 5126127, discloses povidone-iodine solutions stable according to U.S.P. standards and a method for preparing the same. The povidone-iodine solution includes a microbicidal effective amount of available iodine and an alkalinizing agent in an effective amount to maintain the stability of the solution according to U.S.P. standards when said solution is stored in a glass container, the solution being non-buffered. In preferred embodiments, the iodophor solution is a microbicidal povidone-iodine solution for ophthalmic use.

International Patent Application No WO2004/064804, relates to novel povidone-iodine based ophthalmological preparations which also include a viscosity agent. However there is no teaching as to how to prepare the formulation to ensure stability of the product over long-term storage. It is important that products are effective even after storage for 1 - 2 years, and so products need to be shown to be stable over this period.

There is a need for improved, ready to use, povidone-iodine formulations for ophthalmic use packaged for easy administration.

### BRIEF DESCRIPTION OF THE INVENTION

According to the invention we provide a germicidal aqueous formulation, suitable for use in the eye, which has a pH of from 4.0 to 6.5 and which comprises 4.0 to 7.5% w/w of povidone-iodine and a viscosity increasing agent, the formulation buffered with a buffer concentration of 25 mM to 75 mM.

Surprisingly, we have found that in order to ensure that the products are stable, there is a need for a specific buffer concentration. Too high a buffer concentration and the product is unstable with respect to the assay for iodine (the active agent), whilst too low a buffer concentration and the product is unstable with respect to its acidity.

### DETAILED DESCRIPTION OF THE INVENTION

The formulation suitably is isotonic or more preferably hypotonic with respect to the eye. Tonicity may be altered by including in the formulation a tonicity modifier such as sodium chloride.

The formulation suitably is comprised in a sterile single dose unit container for easy administration. Optionally the container will be constructed to protect the contents from light, for example using coloured or opaque materials, and may also be enclosed in further packaging for this purpose.

The formulations according to the invention may be packaged in end use containers of suitable material with which they are compatible and which will not permit the formulations to degrade or the components to permeate through the material. Suitable materials include plastics such as polyethylene terephthalate, polypropylene or preferably high density polyethylene (HDPE), preferably in ampoules made predominantly of high density polyethylene (HDPE), and most preferably in a squeezable HDPE container to provide a single dose, in unit doses ranging from 2 to 100 ml, preferably in unit doses of 5 to 50 ml, and most preferably in unit doses of 5, 10 and 50 ml. Thus containers containing from about 2 to 7 ml, e.g. 5 ml, or about 7 to 12 ml, e.g. 10 ml, or about 25 to 35 ml, e.g 30 ml, or about 45 to 55 ml, e.g 50 ml of the formulation are preferred. The container may be itself either a preformed sterile ampoule that is filled and sealed, or it may be formed, filled and sealed in one process (Blow-Fill-Seal technology).

Multi-dose containers containing the formulation may also be contemplated, but are less preferred.

A container may be fitted with an aerosol spray head and contain a formulation according to the invention whereby the formulation can be delivered as an aerosol spray.

A further aspect of the invention is that the unit dose containers are constructed in such a manner that the seal on the dispensing mouth can be reattached after opening to prevent spillage or contamination.

The formulations, and the containers in which they are packaged, are also preferably such that they can be used directly in the eye.

The content of povidone-iodine in the formulation is preferably in the range 4.0 to 6.0% w/w e.g. 4.5 to 5.5% w/w.

The povidone-iodine may have a K value of 30 to 60 as determined by the USP monograph on povidone-iodine, but preferably has a K value of about 30 to provide an optimal rate of release of iodine to the reservoir and free iodine.

The formulations preferably have a pH between 4.0 and 6.0 e.g. between 5.0 and 6.0.

The formulations contain a viscosity increasing agent. The viscosity increasing agent may be present in a concentration of from 0.05% to 0.5 % w/w, and more preferably from 0.3 to 0.4% w/w. The viscosity increasing agent is typically a polymeric compound, for example carbomers or cellulose based polymers. Preferably the polymer is a carbomer, or carboxymethylcellulose or hydroxypropyl methylcellulose, or more preferably hydroxypropyl methylcellulose (HPMC). Other polymers, e.g. hydroxyethylcellulose, ethyl cellulose, methylcellulose, sodium carboxymethylcellulose or polyvinyl alcohol may also be used. Optionally more than one viscosity increasing agent may be employed.

The HPMC is preferably grade E50, but grades E5 and E15 may also be used. We particularly prefer an HPMC which is sold under the Trade Mark Methocel E50 Premium and which has 7 to 12% hydroxypropoxyl groups and 28 to 30% methoxy groups.

The buffer can be selected from combinations of chemicals that buffer solutions between pH 4.0 and 6.5. For example the buffer may be a citrate buffer, a phosphate buffer or a citrate/phosphate buffer. Preferably the buffer is a citric acid / sodium dihydrogen phosphate buffer or a citric acid / sodium citrate buffer or a disodium hydrogen phosphate / sodium dihydrogen phosphate buffer. The buffer concentration should be in the range of 25-75 mM to provide stability to the formulation with respect to available iodine and pH. Preferably the buffer concentration will be 30-60 mM, preferably 40-55 mM and most preferably 42-51 mM or 40-46 mM e.g. around 43 mM or around 50 mM.

As used herein, "buffer concentration" means the total concentration of the buffering species, for example:
In a citric acid / sodium dihydrogen phosphate buffer - the concentration of citric acid plus the concentration of sodium dihydrogen phosphate;
In a citric acid / sodium citrate buffer - the concentration of citric acid plus the concentration of sodium citrate;
In a disodium hydrogen phosphate / sodium dihydrogen phosphate buffer - the concentration of disodium hydrogen phosphate plus the concentration of sodium dihydrogen phosphate.

The formulation may optionally contain other ingredients, such as a stabiliser e.g. potassium iodate for example in an amount of up to 0.03% w/w e.g. around 0.015% w/w.

One exemplary formulation comprises (for example consist of) povidone-iodine (4.5-5.5 e.g. around 5.0% w/w), hydroxypropylmethylcellulose (0.3-0.4 e.g. around 0.35% w/w), sodium choride (0.38 to 0.42 e.g. around 0.40% w/w), sodium phosphate dibasic anhydrous (0.48 to 0.52 e.g. around 0.50% w/w), citric acid anhydrous (0.14 to 0.16 e.g. around 0.15% w/w) at pH 5.3 to 5.7 e.g. around pH 5.5 containing water qs and characterised by a buffer strength of 40 to 46 mM e.g. around 43 mM.

The percentages and proportions given above for the formulation are those used to make up the formulation.

The formulations preferably have a viscosity such that they can spread easily when they are applied to the eye and insufficient to interfere with any surgical procedure being undertaken.

The formulations preferably have an available iodine content of from 0.43 to 0.66% w/v.

We prefer the formulations to be mildly hypotonic as this has the function of hydrating the cornea in circumstances where tear evaporation is increased, though formulations that are isotonic are suitable.

The formulations are preferably such that the available iodine content remains within +/- 20% of its original value, and preferably within +/- 10% when the formulation is stored for 26 weeks and preferably for 24 months at room temperature.

For the most part the formulations can be made by simple mixing and stirring of the ingredients and where necessary adjustment of the pH, using conventional methods known *per se*. However when carbomer is involved we have surprisingly found that it is desirable to add a solution of povidone-iodine to a solution of carbomer, rather than the reverse.

Thus a method of making a formulation according to the invention and in which the viscosity increasing agent is a carbomer, comprises adding a solution of povidone-iodine to a solution of the carbomer and the other formulation components.

The formulation is intended for administration to the eye and the adnexa of the eye, suitably a human or other mammalian eye, including for paediatric use, in treatment of conditions of the eye.

The formulations of the invention are indicated for use in ocular surgery or other ocular procedures such as intravitreal injection or eye examination. They may be used pre-, peri- or post- operatively. In particular they may be used to irrigate the eye prior to an ocular operation or procedure e.g. to prevent or inhibit endophthalmitis which is a rare, but serious adverse effect, to prevent or inhibit infection and/or for use as an antimicrobial agent. They are preferably applied before the injection of an anaesthetic as such injection may cause some slight haemorrhage and the resulting blood may inhibit the germicidal action of the formulation. In addition they can be used as anti-infective eye drops.

The formulation of the invention may be used as a method for the treatment of infections of the ocular tissue, including the ocular surface and ancillary structures, for example bacterial conjunctivitis, fungal and viral keratitis, and protozoan infections.

As used herein "treatment" embraces therapeutic as well as prophylactic treatment.

The formulations and other aspects of the invention preferably have one or more of the following advantages over existing formulations:
- a ready to use formulation which does not require dilution before use in the eye;
- easy to use single dose container;
- better tolerability (e.g. as evidenced by lower stinging), for example, in a post operative setting;
- a suitable viscosity to allow spread into the various crevices in and around the ocular cavity;
- prolonged on-eye retention time thus enhancing germicidal activity;
- good antimicrobial activity;
- a broad spectrum of antimicrobial activity;
- good long term stability e.g. with respect to maintenance of available iodine content and pH stability.

A suitable application regime is to saturate a sterile cotton-tipped applicator with the formulation to treat lashes and lid margins using one or more applicators per lid; repeating once. The formulation is then used to saturate a sterile sponge or other suitable material to treat lids, brow and cheek in a circular ever-expanding fashion until the entire field is covered; repeating the application three (3) times. While separating the lids, the cornea, conjunctiva and palpebral fornices are irrigated with the formulation using a sterile bulb syringe or by direct dropping from the container. The formulation is left in contact with eye for two minutes, after which time, sterile saline solution in a bulb syringe is used to flush the residual treatment solution from the cornea, conjunctiva, and the palpebral fornices.

### EXAMPLES

The invention, and some of its preferred features, is illustrated, but in no way limited by the following Examples in which the percentages are w/w unless otherwise stated.

### Example 1 - Investigating the stability of 5% PVP-I solutions formulated for ophthalmic use

This example investigated the stability of four povidone-iodine formulations manufactured with different buffer strengths and adjusted to a nominal pH of 5.5. Table 1 shows the composition of the four formulations. The formulations of Examples 1 were made by simple mixing of the ingredients. The ingredients were available for commercial suppliers.

**Table 1. Compositions of PVP-I formulations**

| | | Form A1 | Form A5 | Form 1 | ATC0010 |
|---|---|---|---|---|---|
| Constituent | Function | (% w/w) | (% w/w) | (% w/w) | (% w/w) |
| Povidone-iodine | API | 5 | 5 | 5 | 5 |
| Potassium iodate | Stabiliser | 0 | 0 | 0.015 | 0 |
| Hydroxypropylmethyl-cellulose | Viscosity modifier | 0.35 | 0.35 | 0.35 | 0.35 |
| Sodium chloride | Tonicity modifier | 0.60 | 0.15 | 0.30 | 0.40 |
| Sodium phosphate dibasic anhydrous | Buffer | 0.20 | 0.60 | 0.95 | 0.50 |
| Citric acid anhydrous | Buffer | 0.05 | 0.15 | 0.33 | 0.15 |
| Sodium hydroxide | pH adjust | pH 5.5 | pH 5.5 | pH 5.5 | pH 5.5 |
| Purified water | Solvent | qs | qs | qs | qs |
| | | | | | |
| Buffer strength | | 17 mM | 50 mM | 83 mM | 43 mM |

Stability studies were undertaken on the formulations with storage at 25°C and accelerated conditions of 40°C. Table 2 and 3 shows the results of measuring the available iodine content for each formulation stored at the two temperatures (Table 3 showing accelerated stability testing conditions). The assay for available iodine was a slight modification of the European Pharmacopeia method. As can be seen from Table 3, better stability is evident for Formulation A1, Formulation ATC0010 and A5 compared to Formulation 1. The trend for increasing stability is inversely related to increased buffer strength. This trend also appears to follow from Table 2. Table 4 and 5 show the results of measuring the pH for each formulation stored at the two temperatures (Table 5 showing accelerated stability testing conditions). In this case better pH stability is evident for Formulation 1, Formulation ATC0010 and Formulation A5 compared to Formulation A1. The trend for increasing stability is related to an increase in the buffer strength for the formulations. It is therefore evident that a balance is required in the buffer strength in order to ensure the product is stable with respect to the active agent (iodine) and with respect to pH.

**Table 2. Effect on available iodine content (% w/v) after storage at 25°C**

| Formulation code | Storage time (weeks) | | | | | | |
|---|---|---|---|---|---|---|---|
| | 0 | 2 | 4 | 8 | 13 | 26 | 52 |
| Form A1 | 0.50 | 0.51 | 0.51 | 0.50 (-0%) | 0.49 (-2%) | 0.48 (-4%) | 0.47 (-6%) |
| ATC0010 | 0.54 | 0.54 | 0.53 | 0.54 (0%) | 0.53 (-2%) | 0.50 (0%) | 0.49 (-9%) |
| Form A5 | 0.55 | 0.54 | 0.53 | 0.54 (-2%) | 0.51 (-7%) | 0.52 (-5%) | 0.50 (-9%) |
| Formulation 1 | nd | nd | nd | nd | nd | nd | nd |

| | | | | | | | |
|---|---|---|---|---|---|---|---|
| nd = not determined | | | | | | | |

**Table 3. Effect on available iodine content (% w/v) after storage at 40°C**

| Formulation code | Storage time (weeks) | | | | | |
|---|---|---|---|---|---|---|
| | 0 | 2 | 4 | 8 | 13 | 26 |
| Form A1 | 0.50 | 0.48 | 0.48 | 0.47 (-6%) | 0.46 (-8%) | 0.43 (-14%) |
| ATC0010 | 0.54 | 0.52 | 0.50 | 0.49% (-9%) | 0.47 (-13%) | 0.45 (-17%) |
| Form A5 | 0.55 | 0.52 | 0.51 | 0.50 (-9%) | 0.49 (-11%) | 0.47 (-15%) |
| Formulation 1 | 0.56 | 0.52 | 0.51 | (-11%) | nd | nd |

| | | | | | | |
|---|---|---|---|---|---|---|
| nd = not determined | | | | | | |

**Table 4. Effect on pH after storage at 25°C**

| Formulation code | Storage time (weeks) | | | | | | |
|---|---|---|---|---|---|---|---|
| | 0 | 2 | 4 | 8 | 13 | 26 | 52 |
| Form A1 | 5.38 | 5.31 | 5.16 | 5.00 (-7%) | 4.94 (-8%) | 4.80 (-11%) | 4.60 (-15%) |
| ATC0010 | 5.67 | 5.51 | 5.51 | 5.49 (-3%) | 5.42 (-4%) | 5.33 (-6%) | 5.16 (-9%) |
| Form A5 | 5.46 | 5.47 | 5.33 | 5.20 (-5%) | 5.30 (-3%) | 5.08 (-7%) | 5.06 (-7%) |
| Formulation 1 | nd | nd | nd | nd | nd | nd | nd |

| | | | | | | | |
|---|---|---|---|---|---|---|---|
| nd = not determined | | | | | | | |

**Table 5. Effect on pH after storage at 40°C**

| Formulation code | Storage time (weeks) | | | | | |
|---|---|---|---|---|---|---|
| | 0 | 2 | 4 | 8 | 13 | 26 |
| Form A1 | 5.38 | 4.89 | 4.81 | 4.61 (-14%) | 4.41 (-18%) | 4.21 (-22%) |
| ATC0010 | 5.67 | 5.47 | 5.41 | 5.28 (-7%) | 5.15 (-9%) | 4.96 (-13%) |
| Form A5 | 5.46 | 5.22 | 5.03 | 4.95 (-9%) | 4.98 (-9%) | 4.69 (-14%) |
| Formulation 1 | 5.51 | 5.44 | 5.46 | 5.48 (-1%) | nd | nd |

| | | | | | | |
|---|---|---|---|---|---|---|
| nd = not determined | | | | | | |

### Example 2 - Assessment of Antimicrobial Activity for Water Miscible Compounds Using a Time-Kill Procedure.

The microbiological efficacy of formulation ATC0010 was assessed according to ASTM E2783: Standard Test Method for Assessment of Antimicrobial Activity for Water Miscible Compounds Using a Time-Kill Procedure. An aliquot of the test material ATC0010 is brought into contact with a known population of test organisms for specified periods of time. The activity of the test material is quenched at specified sampling intervals (1, 2, 10 and 30 min) with the appropriate neutralizing technique. The test material is neutralized at the sampling time and the surviving microorganisms enumerated. The percent and log₁₀ reduction, from an initial microbial population is calculated.

Table 6 shows the results against 5 common organisms found in or around the eye.

**Table 6. Time-kill antibacterial efficacy of ATC0010 against common ocular organisms**

| Test organism | Time | Mean count /ml | Log count | Log reduction | % reduction |
|---|---|---|---|---|---|
| *Pseudomonas aeruginosa* ATCC 9027 | 0 | 3000000 | 6.48 | 0.00 | 0 |
| | 1 min | 10 | 1.00 | >5.48 | 99.99966667 |
| | 2 min | 10 | 1.00 | >5.48 | 99.99966667 |
| | 10 min | 10 | 1.00 | >5.48 | 99.99966667 |
| | 30 min | 10 | 1.00 | >5.48 | 99.99966667 |
| *Staphylococcus aureus,* ATCC 6538 | 0 | 3190000 | 6.50 | 0.00 | 0 |
| | 1 min | 10 | 1.00 | >5.50 | 99.99968652 |
| | 2 min | 10 | 1.00 | >5.50 | 99.99968652 |
| | 10 min | 10 | 1.00 | >5.50 | 99.99968652 |
| | 30 min | 10 | 1.00 | >5.50 | 99.99968652 |
| *Staphylococcus epidermidis* Tu3298 | 0 | 2140000 | 6.33 | 0.00 | 0 |
| | 1 min | 151167 | 5.18 | 1.19 | 92.93613707 |
| | 2 min | 1947 | 3.29 | 3.12 | 99.90903427 |
| | 10 min | 10 | 1.00 | >5.33 | 99.99953271 |
| | 30 min | 10 | 1.00 | >5.33 | 99.99953271 |
| *Staphylococcus aureus,* MRSA blood stream infection | 0 | 2560000 | 6.41 | 0.00 | 0 |
| | 1 min | 41167 | 4.61 | 1.85 | 98.39192708 |
| | 2 min | 748 | 2.87 | 3.92 | 99.97076823 |
| | 10 min | 10 | 1.00 | >5.41 | 99.99960938 |
| | 30 min | 10 | 1.00 | >5.41 | 99.99960938 |
| *Streptococcus pneumoniae corneal abscess* | 0 | 1130000 | 6.05 | 0.00 | 0 |
| | 1 min | 10 | 1.00 | >5.05 | 99.99911504 |
| | 2 min | 10 | 1.00 | >5.05 | 99.99911504 |
| | 10 min | 10 | 1.00 | >5.05 | 99.99911504 |
| | 30 min | 10 | 1.00 | >5.05 | 99.99911504 |

As the results show, ATC0010 afforded a rapid kill against all organisms within 10 minutes.

The foregoing broadly describes the present invention, without limitation. Variations and modifications as will be readily apparent to those of ordinary skill in this art are intended to be included within the scope of this application and subsequent patents.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation, the following claims:
The listing or discussion of an apparently prior-published document in this specification should not necessarily be taken as an acknowledgement that the document is part of the state of the art or is common general knowledge.

## Claims

1. A germicidal aqueous formulation, suitable for use in the eye, which has a pH of from 4.0 to 6.5 and which comprises 4.0 to 7.5% w/w of povidone-iodine and a viscosity increasing agent, the formulation buffered with a buffer concentration of 25 mM to 75 mM.

2. A formulation according to claim 1 wherein, independently or in any combination:
the content of povidone-iodine is 4.0 to 6.0% w/w; and/or
the formulation has a pH between 4.0 and 6.0, for example between 5.0 and 6.0; and/or
the formulation contains a viscosity increasing agent present in a concentration of from 0.05% to 0.5 % w/w, for example from 0.3% to 0.4 % w/w.

3. A formulation according to claim 1 or claim 2 wherein the viscosity increasing agent is a polymeric compound selected from hydroxypropyl methylcellulose, carboxymethylcellulose, carbomers, hydroxyethylcellulose, ethyl cellulose, methylcellulose, sodium carboxymethylcellulose and polyvinyl alcohol.

4. A formulation according to any one of claims 1 to 3 where the buffer is a citric / citrate buffer, or a citric / phosphate buffer or a phosphate buffer.

5. A formulation according to any one of claims 1 to 4 where the available iodine content ranges from 0.43 to 0.66% w/v.

6. A formulation according to any one of the preceding claims which is isotonic or hypotonic with respect to the eye.

7. A formulation according to any one of the preceding claims such that the available iodine content remains within +/- 20% of its original value when the formulation is stored for 26 weeks at room temperature.

8. A formulation according to claim 7 such that the available iodine content remains within +/- 10% of its original value when the formulation is stored for 26 weeks at room temperature.

9. A method of making a formulation according to any one of claims 1 to 8 in which the viscosity increasing agent is a carbomer, which comprises adding a solution of povidone-iodine to a solution of the carbomer and the other formulation components.

10. A sterile single dose unit container comprising a formulation according to any one of claims 1 to 8.

11. A single dose unit container according to claim 10 which is an ampoule made of polyethylene terephthalate, polypropylene or high density polyethylene, for example a high density polyethylene squeezable ampoule.

12. A single dose unit container according to claim 10 or claim 11 which contains from 2 to 7 ml of the formulation; or which contains from 7 to 12 ml of the formulation; or which contains from 25 to 35 ml of the formulation.

13. A sterile single dose unit container according to any one of claims 10 to 12 where the unit dose container is constructed in such a manner that the seal on the dispensing mouth can be reattached after opening to prevent spillage or contamination.

14. A single dose unit container fitted with an aerosol spray head and containing a formulation according to any one of claims 1 to 8 whereby the formulation can be delivered as an aerosol spray.

15. A formulation according to any one of claims 1 to 8 for use by administration topically to the eye or adnexa of the eye in treatment of conditions of the eye; or
for use by administration topically to the eye or adnexa of the eye in connection with ocular surgery (pre-, peri- or post- operatively) or other ocular procedures such as intravitreal injection or eye examination; or
for use in irrigating an eye prior to an ocular operation or procedure; or
for use by administration as eye drops as an anti-infective agent; or
for use in treating infections of the ocular tissue.

## Patentansprüche

1. Germizide wässrige, für die Anwendung im Auge geeignete Formulierung mit einem pH-Wert von 4,0 bis 6,5, welche 4,0 bis 7,5 Gew.-% Povidon-Iod und ein viskositätserhöhendes Mittel umfasst, wobei die Formulierung mit einer Pufferkonzentration von 25 mM bis 75 mM gepuffert ist.

2. Formulierung nach Anspruch 1, wobei, unabhängig oder in beliebiger Kombination:
der Gehalt an Povidon-Iod 4,0 bis 6,0 Gew.-% beträgt und/oder
die Formulierung einen pH-Wert zwischen 4,0 und 6,0, zum Beispiel zwischen 5,0 und 6,0, aufweist und/oder
die Formulierung ein viskositätserhöhendes Mittel enthält, welches in einer Konzentration von 0,05 Gew.-% bis 0,5 Gew.-%, zum Beispiel von 0,3 Gew.-% bis 0,4 Gew.-%, vorliegt.

3. Formulierung nach Anspruch 1 oder 2, wobei es sich bei dem viskositätserhöhenden Mittel um eine polymere Verbindung ausgewählt aus Hydroxypropylmethylcellulose, Carboxymethylcellulose, Carbomeren, Hydroxyethylcellulose, Ethylcellulose, Methylcellulose, Natriumcarboxymethylcellulose und Polyvinylalkohol handelt.

4. Formulierung nach einem der Ansprüche 1 bis 3, wobei es sich bei dem Puffer um einen Citronensäure-Citrat-Puffer oder einen Citronensäure-Phosphat-Puffer oder einen Phosphatpuffer handelt.

5. Formulierung nach einem der Ansprüche 1 bis 4, wobei der verfügbare Iodgehalt im Bereich von 0,43 bis 0,66% (w/v) liegt.

6. Formulierung nach einem der vorhergehenden Ansprüche, welche, bezogen auf das Auge, isoton oder hypoton ist.

7. Formulierung nach einem der vorhergehenden Ansprüche, die so beschaffen ist, dass der verfügbare Iodgehalt bei einer 26-wöchigen Lagerung der Formulierung bei Raumtemperatur innerhalb +/- 20% seines ursprünglichen Wertes bleibt.

8. Formulierung nach Anspruch 7, die so beschaffen ist, dass der verfügbare Iodgehalt bei einer 26-wöchigen Lagerung der Formulierung bei Raumtemperatur innerhalb von +/- 10% seines ursprünglichen Wertes bleibt.

9. Verfahren zur Herstellung einer Formulierung nach einem der Ansprüche 1 bis 8, wobei es sich bei dem viskositätserhöhenden Mittel um ein Carbomer handelt, welches die Zugabe einer Lösung von Povidon-Iod zu einer Lösung des Carbomers und der anderen Formulierungskomponenten umfasst.

10. Steriler Einzeldosiseinheitsbehälter, umfassend eine Formulierung nach einem der Ansprüche 1 bis 8.

11. Einzeldosiseinheitsbehälter nach Anspruch 10, bei dem es sich um eine aus Polyethylenterephthalat, Polypropylen oder hochdichtem Polyethylen hergestellte Ampulle handelt, zum Beispiel eine zusammendrückbare Ampulle aus hochdichtem Polyethylen.

12. Einzeldosiseinheitsbehälter nach Anspruch 10 oder 11, welcher 2 bis 7 ml der Formulierung oder 7 bis 12 ml der Formulierung oder 25 bis 35 ml der Formulierung enthält.

13. Steriler Einzeldosiseinheitsbehälter nach einem der Ansprüche 10 bis 12, wobei der Einheitsdosisbehälter so konstruiert ist, dass die Versiegelung auf der Ausgabeöffnung zum Verhindern eines Verschüttens oder einer Verunreinigung nach dem Öffnen wieder angebracht werden kann.

14. Einzeldosiseinheitsbehälter, ausgestattet mit einem Aerosolsprühkopf und enthaltend eine Formulierung nach einem der Ansprüche 1 bis 8, wobei die Formulierung als Aerosolspray verabreicht werden kann.

15. Formulierung nach einem der Ansprüche 1 bis 8 zur Verwendung durch die topische Verabreichung an das Auge oder Augenanhangsgebilde bei einer Behandlung von Augenleiden oder
zur Verwendung durch topische Verabreichung an das Auge oder Augenanhangsgebilde in Zusammenhang mit Augenoperationen (prä-, peri- oder postoperativ) oder anderen Eingriffen am Auge wie einer intravitrealen Injektion oder einer Augenuntersuchung oder
zur Verwendung beim Spülen eines Auges vor einer Augenoperation oder einem Eingriff am Auge oder zur Verwendung durch Verabreichung als Augentropfen als Antiinfektionsmittel oder
zur Verwendung bei der Behandlung von Infektionen des Augengewebes.

## Revendications

1. Formulation aqueuse germicide, adaptée pour utilisation dans l'oeil, qui a un pH de 4,0 à 6,5 et qui comprend de 4,0 à 7,5 % m/m de povidone-iode et un agent augmentant la viscosité, la formulation tamponnée ayant une concentration de tampon de 25 mM à 75 mM.

2. Formulation selon la revendication 1 dans laquelle, indépendamment ou dans une combinaison quelconque :
la teneur de povidone-iode est de 4,0 à 6,0 % m/m ; et/ou
la formulation a un pH entre 4,0 et 6,0, par exemple entre 5,0 et 6,0 ; et/ou
la formulation contient un agent augmentant la viscosité présent à une concentration de 0,05 % à 0,5 % m/m, par exemple de 0,3 % à 0,4 % m/m.

3. Formulation selon la revendication 1 ou la revendication 2 dans laquelle l'agent augmentant la viscosité est un composé polymère choisi parmi l'hydroxypropylméthylcellulose, la carboxyméthylcellulose, des carbomères, l'hydroxyéthylcellulose, l'éthylcellulose, la méthylcellulose, la carboxyméthylcellulose sodique et l'alcool polyvinylique.

4. Formulation selon l'une quelconque des revendications 1 à 3 dans laquelle le tampon est un tampon citrique / citrate, ou un tampon citrique / phosphate ou un tampon phosphate.

5. Formulation selon l'une quelconque des revendications 1 à 4 dans laquelle la teneur en iode disponible est dans la plage de 0,43 à 0,66 % m/v.

6. Formulation selon l'une quelconque des revendications précédentes qui est isotonique ou hypotonique par rapport à l'oeil.

7. Formulation selon l'une quelconque des revendications précédentes **caractérisée en ce que** la teneur en iode disponible reste à +/- 20 % de sa valeur originale lorsque la formulation est conservée pendant 26 semaines à température ambiante.

8. Formulation selon la revendication 7 **caractérisée en ce que** la teneur en iode disponible reste à +/- 10 % de sa valeur originale lorsque la formulation est conservée pendant 26 semaines à température ambiante.

9. Procédé de fabrication d'une formulation selon l'une quelconque des revendications 1 à 8 dans lequel l'agent augmentant la viscosité est un carbomère, qui comprend l'ajout d'une solution de povidone-iode à une solution du carbomère et les autres composants de formulation.

10. Récipient d'unité de dose individuelle stérile comprenant une formulation selon l'une quelconque des revendications 1 à 8.

11. Récipient d'unité de dose individuelle selon la revendication 10 qui est une ampoule constituée de polyéthylène-téréphtalate, polypropylène ou polyéthylène haute densité, par exemple une ampoule compressible en polyéthylène haute densité.

12. Récipient d'unité de dose individuelle selon la revendication 10 ou la revendication 11 qui contient de 2 à 7 ml de la formulation ; ou qui contient de 7 à 12 ml de la formulation ; ou qui contient de 25 à 35 ml de la formulation.

13. Récipient d'unité de dose individuelle stérile selon l'une quelconque des revendications 10 à 12 **caractérisé en ce que** le récipient d'unité de dose est construit de telle manière que le joint d'étanchéité sur l'embouchure de distribution peut être à nouveau attaché après l'ouverture afin d'éviter tout déversement ou contamination.

14. Récipient d'unité de dose individuelle équipé d'une tête de pulvérisation d'aérosol et contenant une formulation selon l'une quelconque des revendications 1 à 8 de sorte que la formulation puisse être administrée sous la forme d'une pulvérisation d'aérosol.

15. Formulation selon l'une quelconque des revendications 1 à 8 pour utilisation par administration par voie topique sur l'oeil ou les annexes de l'oeil dans le traitement d'affections de l'oeil ; ou
pour utilisation par administration topique à l'oeil ou les annexes de l'oeil dans le contexte d'une chirurgie oculaire (pré-, per- ou post-opératoire) ou d'autres procédures oculaires telles qu'une injection intravitréenne ou un examen de l'oeil ; ou
pour utilisation dans l'irrigation d'un oeil avant une opération ou procédure oculaire ; ou
pour utilisation par administration sous la forme de gouttes oculaires en tant qu'agent anti-infectieux ; ou pour utilisation dans le traitement d'infections du tissu oculaire.
